# EUROPEAN PATENT APPLICATION

(11) **EP 4 485 036 A1**
(43) Date of publication of application: **01.01.2025**
(21) Application number: 24182269.1
(22) Date of filing: 14.06.2024
(51) Int. Cl.: G02B 21/00, G02B 27/28, A61B 1/00

(54) **OPTICAL IMAGING SYSTEM AND CORRESPONDING METHOD AND COMPUTER SYSTEM**

(30) Priority: 22.06.2023 DE 102023116504
(71) Applicant: Leica Instruments (Singapore) Pte Ltd, Singapore 608924 (SG)
(72) Inventor: THEMELIS, George, 608924 Singapore (SG)
(74) Representative: 2SPL Patentanwälte PartG mbB

(57) **Abstract**

Examples relate to an optical imaging system, to a corresponding method for an optical imaging system and to a corresponding computer program. The optical imaging system comprises one or more optical imaging sensors (220, 225) for providing imaging sensor data of an object (20) to be imaged. The optical imaging system comprises a diode-based illumination system (230) for emitting at least a first unit of one or more light beams having a first polarization and a second unit of one or more light beams having a second polarization towards the object. The optical imaging system comprises a processing system (210) configured to generate a digital image representation of the object, comprising controlling a contribution of the at least two units of light beams in a digital image representation of the object, by at least one of a) controlling, separately for each of the at least two units, the light emitted by the unit, and b) controlling, separately for each of the at least two polarizations emitted by the at least two units, a contribution of the light having the respective polarization in the digital image representation of the object.

## Description

### Technical field

Examples relate to an optical imaging system, to a corresponding method for an optical imaging system and to a corresponding computer program.

### Background

Cross polarizers are commonly used in medical imaging, particularly in surgical microscopes. In this technique, polarizing filters are used to control the polarization of light passing through the microscope, thereby reducing or eliminating specular reflections that can interfere with the surgeon's view. This enables improved visualization of tissue structures and reduced glare, which can make it easier for the surgeon to see important details during a procedure and perform the surgery with greater accuracy and precision. Additionally, cross polarizers can help to reduce eye strain and improve overall image contrast, allowing the surgeon to work more efficiently and effectively.

There may be a desire for an improved concept for implementing cross polarization in optical imaging systems, such as surgical microscopes.

### Summary

This desire is addressed by the subject-matter of the independent claims.

Various examples of the present disclosure are based on the finding, that many implementations of cross polarization relay on mechanical contraptions to insert or remove the respective polarizers into or from the optical path of the respective optical imaging system they are used in, which limits the speed with which changes can be applied and which requires additional moving parts with consequences in reliability, cost, size, and weight. Moreover, there are limitations with respect to fine adjustment. In the proposed concept, these drawbacks may be overcome by the use of a diode-based illumination system (e.g., a Light-Emitting Diode-based illumination system or a Laser diode-based illumination system) with the capability of (simultaneously) emitting two units of one or more light beam having different polarizations. Using image processing and/or highly precise control of the illumination system, a digital image representation of an object being illuminated can be generated, in which the respective contributions of the light having the two different polarizations are precisely controlled. This enables the use of cross-polarization without requiring a mechanical system for inserting and removing the respective filters from the path and enables a fine-grained control of the amount of specular reflections present in the digital image representation of the object.

Some aspects of the present disclosure relate to an optical imaging system. The optical imaging system comprises one or more optical imaging sensors for providing imaging sensor data of an object to be imaged. The optical imaging system comprises a diode-based illumination system for emitting at least a first unit of one or more light beams having a first polarization and a second unit of one or more light beams having a second polarization towards the object. The optical imaging system comprises a processing system configured to generate a digital image representation of the object. The act of generating the digital of representation of the object comprises controlling a contribution of the at least two units of light beams in a digital image representation of the object, by at least one of a) controlling, separately for each of the at least two units, the light emitted by the unit, and b) controlling, separately for each of the at least two polarizations emitted by the at least two units, a contribution of the light having the respective polarization in the digital image representation of the object. This enables the use of cross-polarization without requiring a mechanical system for inserting and removing the respective filters from the path and enables a fine-grained control of the amount of specular reflections present in the digital image representation of the object.

In some examples, the processing system is configured to control the contribution of the at least two units in the digital image representation by controlling, separately for each of the at least two units, an illumination intensity of the unit. This way, the post-processing required for controlling the contribution of the at least two units can be reduced or minimized. Moreover, this approach is applicable if the at least two units are not sensed separately (e.g., sequentially or by the use of two or more sensors).

Alternatively, or additionally, post-processing can be used to vary the contribution of the two units. In the following, a first approach is presented, in which two separate sensors are used to sense the light having the two different polarizations. For example, the optical imaging system may comprise a first optical imaging sensor for sensing light having the first polarization and a second optical imaging sensor for sensing light having the second polarization. Accordingly, the imaging sensor data may comprise a first component being based on the light having the first polarization and a second component being based on the light having the second polarization. The processing system may be configured to control the contribution of the at least two units of light beams in the digital image representation of the object, by controlling a contribution of the first and second component of the imaging sensor data. This way, the contribution of the two polarizations can be adjusted independently for each viewer, or even offline when reviewing the captured imaging sensor data, at the additional cost and complexity of another optical imaging sensor.

Alternatively, or additionally, time-multiplexing may be used to capture the two polarizations separately. For example, the processing system may be configured to control the contribution of the at least two units in the digital image representation by controlling the illumination system to time-multiplex the at least two units, such that the imaging sensor data comprises a first subset of frames being primarily based on the light having the first polarization and a second subset of frames being primarily based on the light having the second polarization. The processing system may be configured to control the contribution of the at least two units in the digital image representation by controlling a contribution of the first and second subset of frames in the digital image representation of the object. This way, the contribution of the two polarizations can be adjusted independently for each viewer, or even offline when reviewing the captured imaging sensor data. However, the effective frame rate may be halved.

As outlined above, the proposed concept allows for a fine-grained adjustment of the respective contributions. This may be used to vary the amount of specular reflections according to the preference of a user. For example, the processing system may be configured to obtain an input signal, with the input signal indicating a desired amount of specular reflections. The processing system may be configured to control the contribution of the at least two units of light beams in the digital image representation of the object based on the desired amount of specular reflections.

The present disclosure relates to digital imaging, where the object is imaged using an optical imaging sensor and the resulting imaging sensor data is processed to generate a digital image representation of the object. This digital image representation may then be provided via a display device, such as digital oculars, a display screen, or a head-mounted display. For example, the processing circuitry may be configured to generate a display signal based on the digital image representation of the object, and to provide the display signal to a display device.

One potential application of the variation of the contribution of light having different polarizations in a digital image representation is the use in machine-learning based classification. In particular, indicators that are hard to perceive (or even imperceptible) to the human eye can be clearly visible in the digital image representation (or at least the imaging sensor data) and be processed by a machine-learning model to perform a classification. The processing circuitry may be configured to process the digital image representation, using a machine-learning model trained to classify a condition of the object based on the digital image representation, and to provide a result of the classification.

For example, the object may be a surgical site, and the machine-learning model may be trained to classify tissue of the surgical site as pathologic or healthy. For example, tumors may be spotted via the relationship between linearly polarized and cross-polarized light reflected or scattered at the tissue, which can be employed to classify the tissue.

The processing via a machine-learning model is particularly effective if the different contributions of the light having different polarizations can be processed separately by the machine-learning model. For example, the processing system may be configured to generate at least two different digital image representations of the object with at least two different contributions of the at least two units of light beams, and to process the at least two different digital image representation using the machine-learning model.

The proposed concept is not limited to two polarizations (being perpendicular to each other) but can be extended to an arbitrary number of different polarizations. Experiments have shown that the use of four polarizations (two polarizations perpendicular to each other, and another two polarizations filling the gaps (e.g., offset by 45 degrees)) are useful with respect to processing with a machine-learning model, providing an improved accuracy of the classification. For example, the illumination system may be configured to emit four units of one or more light beams having four different polarizations. The processing system may be configured to generate at least four different digital image representations of the object with at least four different contributions of the four units of light beams. The processing system may be configured to process the at least four different digital image representation using the machine-learning model.

In some examples, the optical imaging system may comprise an objective. Illumination diode modules of the diode-based illumination system may be arranged at the objective of the optical imaging system. This may enable a compact construction of the optical imaging system, as light guides for transporting the light and bulky illumination sources can be omitted.

The proposed concept is particularly useful in medical imaging, where the cross-polarization technique is already widely used. For example, the optical imaging system may be one of a (surgical) microscope system, a (surgical) exoscope system and a (surgical) endoscope system.

In the present disclosure, the term "diode-based illumination system" is primarily used for two types of illumination - LED-based illumination, and Laser-based illumination. For example, the diode-based illumination system may be a Light Emitting Diode-, LED, based illumination system or a Laser-based illumination system.

Some aspects of the present disclosure relate to a method for an optical imaging system. The method comprises obtaining imaging sensor data of an object to be imaged of one or more optical imaging sensors. The object is illuminated by a diode-based illumination system emitting at least a first unit of one or more light beams having a first polarization and a second unit of one or more light beams having a second polarization towards the object. The method comprises generating a digital image representation of the object, comprising controlling a contribution of the at least two units of light beams in a digital image representation of the object, by at least one of a) controlling, separately for each of the at least two units, the light emitted by the unit, and b) controlling, separately for each of the at least two polarizations emitted by the at least two units, a contribution of the light having the respective polarization in the digital image representation of the object.

An aspect of the present disclosure relates to computer program with a program code for performing the above method when the computer program may be run on a processor.

### Short description of the Figures

Some examples of apparatuses and/or methods will be described in the following by way of example only, and with reference to the accompanying figures, in which:
- Figs. 1a and 1b: shows an example of the use of cross-polarization;
- Fig. 2a: shows a schematic diagram of an example of an optical imaging system;
- Figs. 2b and 2c: show examples of illumination diode modules arranged at an objective of a microscope;
- Fig. 2d: shows a schematic diagram of an example of a surgical microscope system;
- Fig. 3: shows a flow chart of an example of a method for an optical imaging system; and
- Fig. 4: shows a schematic diagram of an example of a system comprising an optical imaging device and a computer system.

### Detailed Description

Various examples will now be described more fully with reference to the accompanying drawings in which some examples are illustrated. In the figures, the thicknesses of lines, layers and/or regions may be exaggerated for clarity.

Various examples of the present disclosure relate to specular reflection control using polarized LED illumination. The present disclosure proposes an improved optical arrangement for controlling specular reflections, which is improved or optimized for surgical imaging.

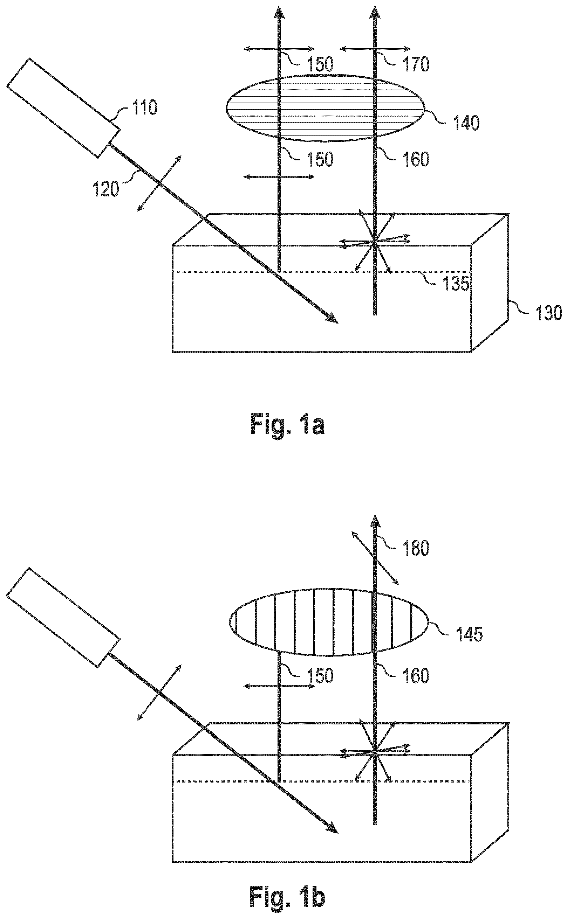

Cross-polarizer imaging is a well-known technique used for a long time in photography and medical imaging. Figs. 1a and 1b shows an example of the use of cross-polarization. In Figs. 1a and 1b, an illumination source 110 emits linearly polarized illumination 120 towards a tissue sample 130. Some of the illumination is reflected at a superficial layer 135 of the tissue sample, resulting in a linearly polarized reflection 150 that is filtered by a linear polarizer 140; 145. In Fig. 1a, the linear polarizer 140 is in copolarized orientation, and the linearly polarized reflection 150 passes through the linear polarizer 140. In Fig. 1b, the linear polarizer 145 is in cross-polarized orientation, and the linearly polarized reflection 150 is blocked by the linear polarizer 145. In addition to the linearly polarized reflection 150, randomly polarized light 160 is reflected by the lower layers of the tissue sample 130. In both cases, a portion 170, 180 of the randomly polarized light 160 passes through the respective linear polarizer 140, 145. Using the linear polarizer in cross-polarized orientation, the linearly polarized reflection, which may include specular reflections, can be blocked.

For a system that can perform imaging in both modes, i.e., with and without specular reflections, the respective polarizers are, in common implementations, moved physically in and out of the optical path of the illumination or detection. This reduces the speed in which changes can be applied and requires additional moving parts with consequences in reliability, cost, size, and weight. Moreover, there are limitations with respect to fine adjustment of specular reflection, e.g., when the user wants to attenuate the specular reflections so that they are at moderate intensity to improve visualization of underlying tissue features.

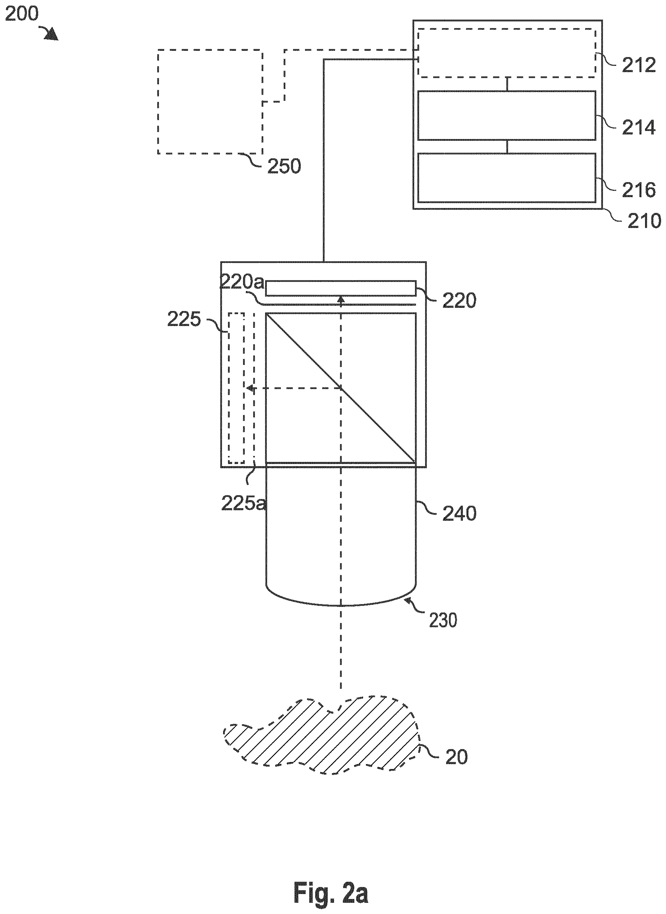

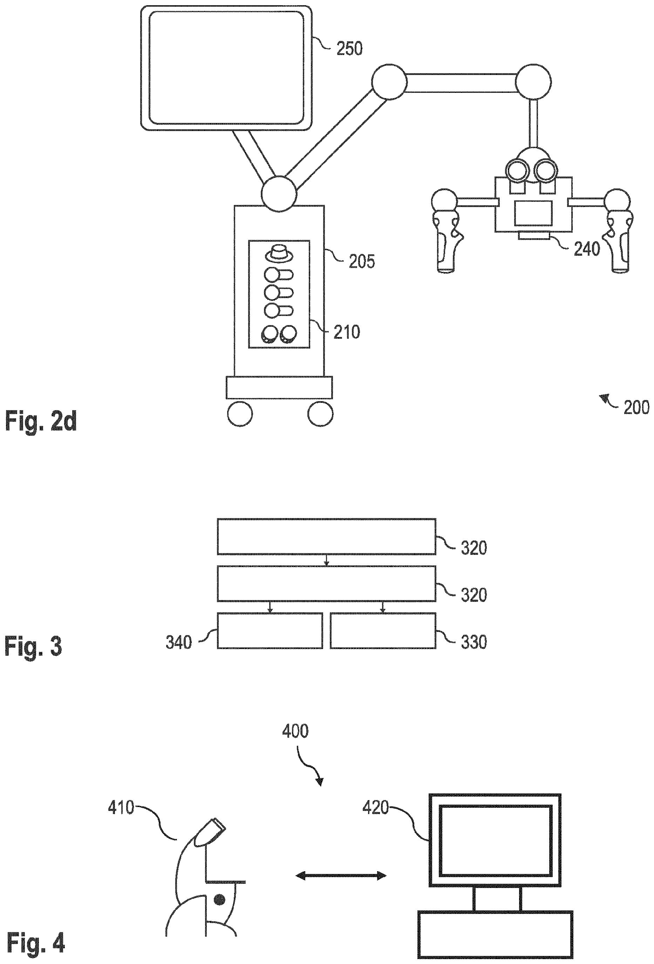

Fig. 2a shows a schematic diagram of an example of an optical imaging system 200. For example, as shown in Fig. 2d, the optical imaging system 200 may be an optical imaging system for use in medical imaging, i.e., a medical or surgical imaging system 200. In particular, the optical imaging system 200 may be one of a (surgical) microscope system, a (surgical) exoscope system and a (surgical) endoscope system, i.e., a microscope system, exoscope system or endoscope system for use during surgical procedures. For example, the respective optical imaging system may comprise an optical imaging device comprising the optical components (sometimes also referred to as "optics carrier"), such as a microscope, an exoscope or an endoscope, and one or more additional components. In the present case, the respective optical imaging system comprises, in addition to the optical imaging device comprising one or more optical imaging sensors 220, 225 for providing imaging sensor data of an object 20 to be imaged, an illumination system 230 and a processing system 210, which will be introduced in more detail in the following.

In general, a microscope is an optical instrument that is suitable for examining objects that are too small to be examined by the human eye (alone). For example, a microscope may provide an optical magnification of a sample, such as an object 20 shown in Fig. 1a. In modern microscopes, the optical magnification is often provided for a camera or an imaging sensor, such as the one or more optical imaging sensors 220; 225. In other words, the microscope may comprise the one or more optical imaging sensors 220; 225. The microscope may further comprise one or more optical magnification components that are used to magnify a view of the objective, such as an objective (i.e., lens). Exoscopes are camera-based imaging systems, and in particular camera-based 3D imaging systems, which are suitable for providing images of surgical sites with high magnification and a large depth of field. Accordingly, an exoscope may comprise the one or more optical imaging sensors 220; 225. Compared to microscopes, which may be used via oculars, exoscopes are only used via display modalities, such as monitor or a head-mounted display. Such devices are usually part, or attached to, a surgical imaging system, such as a surgical microscope system or a surgical exoscope system. An endoscope is a medical device consisting of a long, thin, flexible tube with an optical imaging sensor and light source (i.e., an illumination system) on its tip. Accordingly, an endoscope may comprise the one or more optical imaging sensors 220; 225. An endoscope is used to visualize the internal organs and structures of the body, e.g., without the need for invasive surgery. It is usually inserted through natural openings in the body, such as the mouth, nose, anus, or urethra, and may also be used during surgical procedures.

In addition to the optical components and optical imaging sensor(s) for generating the imaging sensor data, the optical imaging system further comprises the illumination system 230. In particular, the optical imaging system comprises a diode-based illumination system 230. A diode-based illumination system is an illumination system that is based on using at least one diode, such as a Light-Emitting Diode (LED) or a Laser diode, to generate the illumination. In other words, the diode-based illumination system may be a LED based illumination system or a Laser-based illumination system. For example, the diode-based illumination system may comprise diodes, such as Laser diodes, edge emitter diodes, or VCSEL (Vertical Cavity Surface Emitting Laser) diodes.

The diode-based illumination system 230 is suitable for emitting at least a first unit of one or more light beams having a first polarization and a second unit of one or more light beams having a second polarization towards the object. For example, a first portion of the light emitted by the diode-based illumination system may be filtered by a polarizer having the first polarization, and a second portion of the light emitted by the diode-based illumination system may be filtered by (another) polarizer having the second polarization. For example, polarization filters and wire-grid polarizer may be used as polarizer. For example, the diode-based illumination system 230 may comprise a plurality of diodes (LED diodes, Laser diodes etc.), with a first subset of the diodes emitting light through a polarizer having the first polarization and a second subset of the diodes emitting light through a polarizer having the second polarization. For example, the first polarization may be perpendicular (e.g., orthogonal) to the second polarization (in case of linear polarization). Alternatively, if circular polarizers are used, the first polarization may be a clockwise or right-handed circular polarizer, and the second polarization may be a counter-clockwise or left-handed circular polarizer.

However, the proposed concept is not limited to two polarizations. In some examples, four different polarizations may be used. A third and a fourth polarization may be rotated by 45 degrees relative to the first and second polarization, respectively. In case four polarizations are used, a third polarization may be filtered by a polarizer having the third polarization, and a fourth portion of the light emitted by the diode-based illumination system may be filtered by (another) polarizer having the fourth polarization. For example, a third subset of the diodes may emit light through a polarizer having the third polarization and a fourth subset of the diodes emitting light through a polarizer having the fourth polarization. In other words, the respective diodes may each be combined with a polarizer having one of the polarizations.

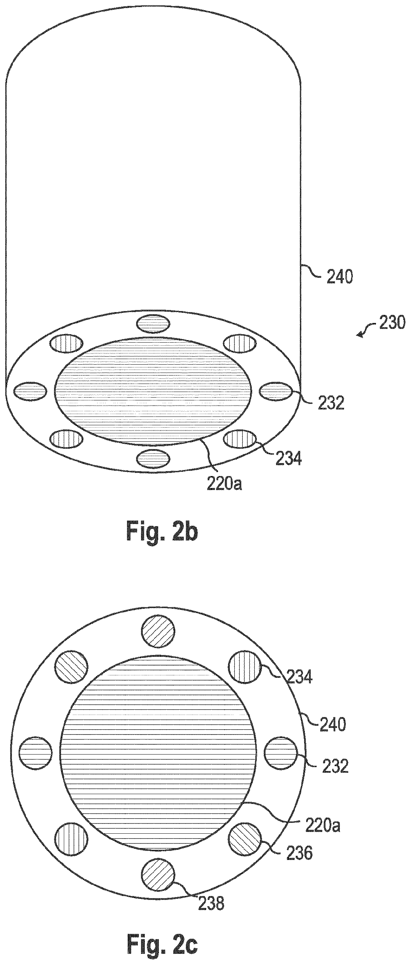

Figs. 2b and 2c show examples of illumination diode modules arranged at an objective 240 of a microscope. In Fig. 2b, a diode-based illumination system for emitting light having two different (linear) polarizations is shown, provided by illumination diode modules 232 (with polarizers having the first polarization) and illumination diode modules 234 (with polarizers having the second polarization). As shown in Figs. 2b and 2c the optical imaging system may comprise an objective 240, and the illumination diode modules 232, 234 of the diode-based illumination system may be arranged at the objective of the optical imaging system (e.g., surrounding the objective). In addition, a polarizer 220a (e.g., having the first or second polarization) may be placed in front of the objective, e.g., between the objective and the object to be imaged. In other words, the light sensed by at least one of the one or more imaging sensors may be filtered by a polarizer being arranged between the respective imaging sensor and the object to be imaged.

In Fig. 2c, a diode-based illumination system for emitting light having four different (linear) polarizations is shown, provided by (first) illumination diode modules 232 (with polarizers having the first polarization), (second) illumination diode modules 234 (with polarizers having the second polarization), (third) illumination diode modules 236 (with polarizers having the third polarization), and (fourth) illumination diode modules 238 (with polarizers having the fourth polarization). For example, each illumination diode module may comprise a diode, e.g., an LED diode or a Laser diode, and a polarizer. In addition, the illumination system 230 may comprise a driver or drivers for driving the diodes of the diode-based illumination system, e.g., each LED diode or Laser diode may be coupled with a driver circuit for driving the respective diode.

As shown in Figs. 2b and 2c, the use of multiple LEDs per polarization arrangement ensures sufficient brightness, and more homogenous illumination with reduced or minimum differences between the illumination groups. For example, the intensity of the LEDs may be adjusted so that regardless of the intensity ratio between the two groups of LEDs, the total amount of illumination is such that the displayed image always has the same brightness.

As shown in Figs. 2b and 2c, the illumination provided by the optical imaging system may comprise LEDs or Laser diodes covered with linear polarizers (shown as illumination modules 132-138. In Fig. 2b, half of them have the polarizer orientation vertical and the other half horizontal. The objective lens is also covered with a linear polarizer 220a aligned with the one half of LEDs. In Fig. 2c, four different polarization orientations are implemented by illumination modules 232-238.

In many cases, the illumination system is used in a continuous or pulse width modulation-based pseudo-continuous operation. In some cases, e.g., if time-multiplexing is employed, the illumination system may be operated in a pulsed configuration, i.e., such that the light emitted by the illumination system is pulsed. For example, light having different polarizations may be emitted (i.e., pulsed) alternatingly.

The optical imaging system further comprises a processing system 210. For example, the processing system 210 may comprise one or more interfaces 212, one or more processors 214 and one or more storage devices 216. For example, the one or more processors 214 may be coupled to the one or more interfaces 212 and to the one or more storage devices 216. For example, the one or more processors may be configured to provide the functionality of the processing system 210, in conjunction with the one or more interfaces (for receiving and transmitting information, such as the imaging sensor data and/or a control signal for controlling the illumination system) and/or the one or more storage devices 216 (for storing information, such as machine-readable instructions and/or a machine-learning model). For example, the processing system may be configured to provide its functionality by the one or more processors executing machine-readable instructions. For example, the processing system may comprise the machine-readable instructions, e.g., within the one or more storage devices.

The proposed concept is based on the use of two or more illumination sources, each with a linear (or circular) polarizer, e.g., one with polarization axis parallel to the imaging polarizer, and the other perpendicular. Figs. 2a to 2d illustrate a surgical microscope utilizing linear polarizers for imaging and illumination. The illumination system comprises at least two groups of diode-based light sources, such as LEDs, e.g., one group with polarizers parallel to the polarization of the imaging channel, and the other group with perpendicular polarization. By adjusting the intensity of the two LED groups, the amount of specular reflections captured by the optical imaging device (e.g., microscope) can be controlled. For example, if the illumination comes only from the LEDs with parallel polarization, the specular reflections may be eliminated, and if the other group with perpendicular polarization illuminate, then the specular reflections are visible. Alternatively, or additionally, if both types of illumination can be captured separately, the contribution of the different types of illumination (i.e., the different units of illumination having different polarizations) can be varied during generation of a digital image representation of the object. Any intensity combination of the two groups/units is possible creating the ability for continuous adjustment of specular reflection intensity.

In the proposed concept, the contribution of the at least two units of light beams can be observed in the digital image representation of the object. In particular, the processing system is configured to generate a digital image representation of the object. This digital image representation may be a 2D- or 3D image of the object that is generated based on the imaging sensor data. To vary the contribution of the at least two units (i.e., of the at least two different types of illumination having different polarizations), two approaches may be used (as alternatives or in combination) - the actual intensity of the illumination may be varied, and/or the composition of the digital image representation based on components related to the different polarizations may be adjusted to the desired contribution. In effect, generating the digital image representation of the object comprises controlling a contribution of the at least two units of light beams in a digital image representation of the object, by at least one of a) controlling, separately for each of the at least two units, the light emitted by the unit, and b) controlling, separately for each of the at least two polarizations emitted by the at least two units, a contribution of the light having the respective polarization in the digital image representation of the object.

Case a) is the more straightforward of the two cases, as, in this case, the actual intensity of the different illumination sources (i.e., diodes) is varied. For example, the processing system may be configured to control the contribution of the at least two units in the digital image representation by controlling, separately for each of the at least two units, an illumination intensity of the unit. This may be done by controlling the illumination intensity of the respective diodes providing the illumination, or by varying a number of diodes contributing to the respective units of light beams.

Alternatively, or additionally, in case b), the contribution of the respective units can be controlled through image processing. For this purpose, the different polarizations may be represented separately in the imaging sensor data. In some examples, this can be achieved by the use of separate sensors for each (or a subset) of the different polarizations. For example, the optical imaging system may comprise a first optical imaging sensor 220 for sensing light having the first polarization and a second optical imaging sensor 225 for sensing light having the second polarization. For example, the first optical imaging sensor 220 may be coupled with a first polarizer 220a for admitting light having the first polarization (and blocking light having a different polarization), and the second optical imaging sensor 220 may be coupled with a second polarizer 225a for admitting light having the second polarization (and blocking light having a different polarization). However, the concept is not limited to two different sensors. For example, four optical imaging sensors may be used to separately sense light having four different polarizations. For example, beam splitters may be used to distribute the light to the two (or four) different optical imaging sensors. For example, the beam splitters may be combined with the respective polarizers being used, e.g., by printing or depositing a wire grid on the respective beam splitter. The resulting imaging sensor data may comprise a first component being based on the light having the first polarization and a second component being based on the light having the second polarization (and third and fourth component, for example). In this case, the processing system may be configured to control the contribution of the at least two units of light beams in the digital image representation of the object, by controlling a contribution of the first and second component of the imaging sensor data in the digital image representation of the object.

In addition, the use of diode-based illumination modules, such as LEDs, with a comparatively low response time allows time multiplexing. Time multiplexing may enable imaging modes such as a very fast sequential capture of images with and without specular reflection, which the users would experience as simultaneous imaging. Time multiplexing may enable imaging modes such as virtually-simultaneous capture of backscattered and specular reflection images, so that the amount of specular reflection can be adjustable offline. Accordingly, time-multiplexing may be used, e.g., in combination with a pulsed operation of the illumination system. For example, the processing system may be configured to control the contribution of the at least two units in the digital image representation by controlling the illumination system to time-multiplex the at least two units (i.e., by pulsing the respective diodes). In effect, the imaging sensor data may comprise a first subset of frames being primarily based on the light having the first polarization and a second subset of frames being primarily based on the light having the second polarization. The processing may be configured to control the contribution of the at least two units in the digital image representation by controlling a contribution of the first and second subset of frames in the digital image representation of the object. This technique may be combined with the above technique, e.g., by using two imaging sensors and time-multiplexing to separately represent four types of polarizer light in the imaging sensor data.

A major application of the proposed concept lies in providing the user (e.g., a surgeon) with a more fine-grained control over how specular reflections are represented in the digital image representation. While specular reflections can result in some details (on the surface) being hard to perceive (as the specular reflection is perceived as bright spot), they help the user with respect to three-dimensional perception, which is an important factor during surgery. Using the proposed concept, the user can continuously adjust the intensity of specular reflection. For example, the user may use an input device, such as a rotary dial, to continuously adjust the amount of specular reflections. For example, the processing system may be configured to obtain an input signal (from an input device, such a rotary dial, a touch screen, or a voice control interface), with the input signal indicating a desired amount of specular reflections (as selected by the user). The processing system may be configured to control the contribution of the at least two units of light beams in the digital image representation of the object based on the desired amount of specular reflections. The adjustment may be (near-) instantaneous, as the LEDs (or other diode-based light sources) have a very fast response. Since this implementation has no moving parts, the optical imaging system becomes more reliable, faster, smaller, and less expensive.

To observe the digital image representation, the digital image representation may be output via a display device, such as the display device 250 of the optical imaging system. For example, the processing circuitry may be configured to generate a display signal based on the digital image representation of the object, and to provide the display signal to the display device. For example, the display signal may be a signal for driving (e.g., controlling) the display device 250. For example, the display signal may comprise video data and/or control instructions for driving the display device. For example, the display signal may be provided via one of the one or more interfaces 212 of the system. Accordingly, the processing system 210 may comprise a video interface 212 that is suitable for providing the video signal to the display device 250. The display signal comprises the digital image representation of the object. For example, the display device 250 may be a display screen for showing two-dimensional or three-dimensional content, or a head-mounted display, or ocular displays.

In addition (or as an alternative) to outputting the digital image representation via a display device, machine-learning may be used to process the digital image representation. For example, the processing circuitry may be configured to process the digital image representation, using a machine-learning model trained to classify a condition of the object based on the digital image representation, and to provide a result of the classification.

In the following, a short introduction to machine-learning is given, followed by application of machine-learning on the processing of the digital image representation.

Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g., words or sentences) and associated training content information (e.g., labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g., sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e., each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g., a classification algorithm, a regression algorithm or a similarity learning algorithm. Classification algorithms may be used when the outputs are restricted to a limited set of values (categorical variables), i.e., the input is classified to one of the limited set of values.

In the present case, the machine-learning model is used for classifying a condition of the object shown in the digital image representation, i.e., for performing an image classification on at least a portion of the digital image representation. Accordingly, the machine-learning model may be trained, using supervised learning, as a classifier. To give an example, the object may be a surgical site, and the machine-learning model may be trained to classify tissue of the surgical site as pathologic or healthy. In this case, the machine-learning model may be trained with a plurality of images of surgical sites (i.e., digital image representations of the surgical site) as training input data, and a plurality of corresponding classifications ("healthy", "pathologic") of the plurality of images of surgical sites as desired output. Alternatively, a more complex setup may be used, in which the desired output is an image map of portions of the respective images of the surgical site being classified as healthy or pathologic. While, in the former case, the output of the machine-learning model is a binary classification ("healthy", "pathologic", e.g., combined with a confidence value), in the latter case, the output of the machine-learning model is an image map with a classification of portions of the respective images input into the machine-learning model.

However, the proposed concept is not limited to the above-referenced classification task. Other types of classification may also be possible ("blood vessel", "soft tissue", "bone"), also outside the field of surgical imaging.

While the processing of the digital image representation is applicable to single digital image representations, in some cases, the reliability of the classification can be improved by generating different digital image representations (which different contributions of the at least two units of light beams, and therefore of the light having different polarizations), and processing them via different inputs of the machine-learning model. For example, the processing system may be configured to generate at least two different digital image representations of the object with at least two different contributions of the at least two units of light beams, and to process the at least two different digital image representation using the machine-learning model. For example, one digital image representation with specular reflections and one digital image representation without specular reflections may be processed by the machine-learning model. In this case, the machine-learning model may be set up to accept at least two separate digital image representations (e.g., surgical images) as input, and training of the machine-learning model may be performed using the at least two separate digital image representations per sample of training input data (and a corresponding classification as desired output).

The proposed concept is not limited to two different image representations. For example, the illumination system may be configured to emit four units of one or more light beams having four different polarizations, e.g., two perpendicular to each other, and two offset by 45 degrees to the other two. The processing system may be configured to generate at least four different digital image representations of the object with at least four different contributions of the four units of light beams, and to process the at least four different digital image representation using the machine-learning model. This may further improve the classification accuracy of the machine-learning based classification.

While the principle of the proposed concept was explained with white LEDs, the proposed concept may be implemented with non-white LEDs or combination of LEDs. For example, an application may require multispectral illumination, which is achieved by simultaneously illuminating by a set of LEDs. Instead of LED, any type of (diode-based) light source may be used. For example, lasers typically exhibit very quick response time.

While the proposed concept is applicable to a wide range of different optical imaging systems, it is particularly useful in surgical imaging. Fig. 2d shows a schematic diagram of an example of a surgical microscope system, as an example of the optical imaging system. The surgical imaging system comprises an optics carrier (indicated by the objective 240 of the optical imaging device), a base unit 205 (comprising the processing system 210), a display device 250 (and digital oculars shown at the optics carrier), and an arm for attaching the optics carrier and the display device 250 to the base unit 205.

In various examples of the proposed surgical microscope system, one or more optical imaging sensors 220; 225 are used to provide the imaging sensor data. Accordingly, the one or more optical imaging sensors are configured to generate the respective imaging sensor data. For example, the one or more optical imaging sensors 220; 225 of the optical imaging system (e.g., of the optical imaging system) may comprise or be APS (Active Pixel Sensor) - or a CCD (Charge-Coupled-Device)-based imaging sensors. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensors by a circuitry of the imaging sensors to perform the imaging. The processing system 210 may be configured to obtain (i.e., receive or read out) the imaging sensor data from the respective optical imaging sensor. The imaging sensor data may be obtained by receiving the imaging sensor data from the respective optical imaging sensor (e.g., via the interface 212), by reading the imaging sensor data out from a memory of the respective optical imaging sensor (e.g., via the interface 212), or by reading the imaging sensor data from a storage device 216 of the processing system 210, e.g., after the imaging sensor data has been written to the storage device 216 by the respective optical imaging sensor or by another system or processor.

The one or more interfaces 212 of the processing system 210 may correspond to one or more inputs and/or outputs for receiving and/or transmitting information, which may be in digital (bit) values according to a specified code, within a module, between modules or between modules of different entities. For example, the one or more interfaces 212 may comprise interface circuitry configured to receive and/or transmit information. The one or more processors 214 of the processing system 210 may be implemented using one or more processing units, one or more processing devices, any means for processing, such as a processor, a computer or a programmable hardware component being operable with accordingly adapted software. In other words, the described function of the one or more processors 214 may as well be implemented in software, which is then executed on one or more programmable hardware components. Such hardware components may comprise a general-purpose processor, a Digital Signal Processor (DSP), a micro-controller, etc. The one or more storage devices 216 of the processing system 210 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the optical imaging system 200 and processing system 210 are mentioned in connection with the proposed concept or one or more examples described above or below (e.g., Fig. 1a to 1b, 3 to 4). The optical imaging system 200 and processing system 210 may comprise one or more additional optional features corresponding to one or more aspects of the proposed concept, or one or more examples described above or below.

Fig. 3 shows a flow chart of an example of a corresponding method for an optical imaging system, e.g., for the optical imaging system 200 introduced in connection with Figs. 2a to 2d. The method comprises obtaining 310 imaging sensor data of an object to be imaged of one or more optical imaging sensors. The object is illuminated by a diode-based illumination system emitting at least a first unit of one or more light beams having a first polarization and a second unit of one or more light beams having a second polarization towards the object. The method comprises generating 320 a digital image representation of the object. Generating the digital image representation comprises controlling a contribution of the at least two units of light beams in a digital image representation of the object, by at least one of a) controlling 330, separately for each of the at least two units, the light emitted by the unit, and b) controlling 340, separately for each of the at least two polarizations emitted by the at least two units, a contribution of the light having the respective polarization in the digital image representation of the object.

More details of the method are given with respect to the optical imaging system 200 and processing system 210 of Figs. 2a to 2d. Features introduced in connection with the optical imaging system 200 and processing system 210 may likewise be included in the corresponding method of Fig. 3.

More details and aspects of the method are mentioned in connection with the proposed concept, or one or more examples described above or below (e.g., Fig. 1a to 2d, 4). The method may comprise one or more additional optional features corresponding to one or more aspects of the proposed concept, or one or more examples described above or below.

Some embodiments relate to an optical imaging device, such as a microscope, an endoscope or an exoscope, comprising a system as described in connection with one or more of the Figs. 1a to 3. Alternatively, an optical imaging device may be part of or connected to a system as described in connection with one or more of the Figs. 1a to 3. Fig. 4 shows a schematic illustration of a system 400 configured to perform a method described herein. The system 400 comprises an optical imaging device 410, such as a microscope, an endoscope or an exoscope, and a computer system 420. The optical imaging device 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera, or an illumination unit, etc. of the optical imaging device 410.

The computer system 420 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random-access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer, or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine-readable carrier.

Other embodiments comprise the computer program for performing one of the methods described herein, stored on a machine-readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device, or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

In the following, some additional background is given on machine-learning.

Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied, and an unsupervised learning algorithm may be used to find structure in the input data (e.g., by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e., outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g., a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g., be used to store, manipulate, or apply the knowledge.

Machine-learning algorithms are usually based on a machine-learning model. In other words, the term "machine-learning algorithm" may denote a set of instructions that may be used to create, train, or use a machine-learning model. The term "machine-learning model" may denote a data structure and/or set of rules that represents the learned knowledge (e.g., based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g., of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e., to achieve a desired output for a given input. For example, the machine-learning model may be a deep neural network, i.e., an artificial neural network having at least one hidden layer (of hidden nodes).

Alternatively, the machine-learning model may be a support vector machine, a random forest model or a gradient boosting model. Support vector machines (i.e., support vector networks) are supervised learning models with associated learning algorithms that may be used to analyze data (e.g., in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of reference Signs

- 20: Object
- 110: Illumination source
- 120: Polarizer light
- 130: Tissue
- 135: Superficial layer
- 140: Linear polarizer
- 145: Linear polarizer
- 150: Linearly polarizer reflection
- 160: Randomly polarizer light
- 170: Portion of randomly polarized light
- 180: Portion of randomly polarized light
- 200: Optical imaging system
- 205: Base unit
- 210: Processing system
- 212: Interface
- 214: Processor
- 216: Storage device
- 220: Optical imaging sensor
- 220a: Polarizer
- 225: Optical imaging sensor
- 225a: Polarizer
- 230: Illumination system
- 240: Objective
- 250: Display device
- 310: Obtaining imaging sensor data
- 320: Generating a digital image representation
- 330: Controlling light emitted by unit of light beams
- 340: Controlling a contribution of light
- 400: System
- 410: Optical imaging device
- 420: Computer system

## Claims

1. An optical imaging system comprising:
one or more optical imaging sensors (220; 225) for providing imaging sensor data of an object (20) to be imaged;
a diode-based illumination system (230) for emitting at least a first unit of one or more light beams having a first polarization and a second unit of one or more light beams having a second polarization towards the object;
a processing system (210) configured to:
generate a digital image representation of the object, comprising controlling a contribution of the at least two units of light beams in a digital image representation of the object, by at least one of
a) controlling, separately for each of the at least two units, the light emitted by the unit, and
b) controlling, separately for each of the at least two polarizations emitted by the at least two units, a contribution of the light having the respective polarization in the digital image representation of the object.

2. The optical imaging system according to claim 1, wherein the processing system is configured to control the contribution of the at least two units in the digital image representation by controlling, separately for each of the at least two units, an illumination intensity of the unit.

3. The optical imaging system according to one of the claims 1 or 2, wherein the optical imaging system comprises a first optical imaging sensor (220) for sensing light having the first polarization and a second optical imaging sensor (225) for sensing light having the second polarization, the imaging sensor data comprising a first component being based on the light having the first polarization and a second component being based on the light having the second polarization, the processing system being configured to control the contribution of the at least two units of light beams in the digital image representation of the object, by controlling a contribution of the first and second component of the imaging sensor data.

4. The optical imaging system according to claim 1, wherein the processing system is configured to control the contribution of the at least two units in the digital image representation by controlling the illumination system to time-multiplex the at least two units, such that the imaging sensor data comprises a first subset of frames being primarily based on the light having the first polarization and a second subset of frames being primarily based on the light having the second polarization, and by controlling a contribution of the first and second subset of frames in the digital image representation of the object.

5. The optical imaging system according to one of the claims 1 to 4, wherein the processing system is configured to obtain an input signal, the input signal indicating a desired amount of specular reflections, and to control the contribution of the at least two units of light beams in the digital image representation of the object based on the desired amount of specular reflections.

6. The optical imaging system according to one of the claims 1 to 5, wherein the processing circuitry is configured to generate a display signal based on the digital image representation of the object, and to provide the display signal to a display device.

7. The optical imaging system according to one of the claims 1 to 6, wherein the processing circuitry is configured to process the digital image representation, using a machine-learning model trained to classify a condition of the object based on the digital image representation, and to provide a result of the classification.

8. The optical imaging system according to claim 7, wherein the object is a surgical site, and the machine-learning model is trained to classify tissue of the surgical site as pathologic or healthy.

9. The optical imaging system according to one of the claims 7 or 8, wherein the processing system is configured to generate at least two different digital image representations of the object with at least two different contributions of the at least two units of light beams, and to process the at least two different digital image representation using the machine-learning model.

10. The optical imaging system according to claim 9, wherein the illumination system is configured to emit four units of one or more light beams having four different polarizations, wherein the processing system is configured to generate at least four different digital image representations of the object with at least four different contributions of the four units of light beams, and to process the at least four different digital image representation using the machine-learning model.

11. The optical imaging system according to one of the claims 1 to 10, wherein the optical imaging system comprises an objective (240), wherein illumination diode modules (232; 234; 236; 238) of the diode-based illumination system are arranged at the objective of the optical imaging system.

12. The optical imaging system according to one of the claims 1 to 11, wherein the optical imaging system is one of a microscope system, an exoscope system and an endoscope system.

13. The optional imaging system according to one of the claims 1 to 12, wherein the diode-based illumination system is a Light Emitting Diode-, LED, based illumination system or a Laser-based illumination system.

14. A method for an optical imaging system, the method comprising:
obtaining (310) imaging sensor data of an object to be imaged of one or more optical imaging sensors, the object being illuminated by a diode-based illumination system emitting at least a first unit of one or more light beams having a first polarization and a second unit of one or more light beams having a second polarization towards the obj ect;
generating (320) a digital image representation of the object, comprising controlling a contribution of the at least two units of light beams in a digital image representation of the object, by at least one of
a) controlling (330), separately for each of the at least two units, the light emitted by the unit, and
b) controlling (340), separately for each of the at least two polarizations emitted by the at least two units, a contribution of the light having the respective polarization in the digital image representation of the object.

15. Computer program with a program code for performing the method according to claim 14 when the computer program is run on a processor.
